(19) Europäisches Patentamt
European Patent Office
Office européen des brevets

(11) **EP 4 410 189 A1**

(12) **EUROPEAN PATENT APPLICATION**

(43) Date of publication:
**07.08.2024 Bulletin 2024/32**

(21) Application number: **23154691.2**

(22) Date of filing: **02.02.2023**

(51) International Patent Classification (IPC):
**A61B 5/024** $^{(2006.01)}$   **A61B 5/055** $^{(2006.01)}$
**A61B 5/00** $^{(2006.01)}$   A61B 5/1455 $^{(2006.01)}$

(52) Cooperative Patent Classification (CPC):
**A61B 5/7203; A61B 5/02416; A61B 5/055;
G01R 33/283; G01R 33/5673; G06T 7/0016;
G16H 50/00;** A61B 5/0077; A61B 5/14551;
A61B 2576/00; G06T 2207/30076

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **WEISS, Steffen
Eindhoven (NL)**

• **DEN BRINKER, Albertus Cornelis
Eindhoven (NL)**
• **WUELBERN, Jan Hendrik
5656AG Eindhoven (NL)**
• **DER SARKISSIAN, Henri Henroutune
Eindhoven (NL)**

(74) Representative: **Philips Intellectual Property &
Standards
High Tech Campus 52
5656 AG Eindhoven (NL)**

(54) **IDENTIFICATION AND MITIGATION OF FLICKER MIMICKING PPG SIGNAL**

(57) Disclosed herein is a method comprising: acquiring a first series of images of a subject using an optical imaging system at a first frame rate; determining first clusters of pixels within the first series of images; determining that a flicker signal is present in one or more of the first clusters of pixels; performing at least one of performing a combined weighting of the first clusters pixels for determining a first photoplethysmography signal, wherein the weighting is performed to mitigate or remove a contribution of the flicker signal; or acquiring a second series of images with a second frame rate in order to prevent the flicker signal; determining second clusters of pixels within the second series of images; and combining the second clusters pixels for determining a second photoplethysmography signal; and deriving vital sign information of the subject from the first photoplethysmography signal and/or second photoplethysmography signal.

Acquiring a first series of images of a subject at a first frame rate using the optical imaging system

Determining first clusters of pixels within the first series of images

One or more of the first clusters of pixels have flicker signals?

Yes

207 and/or 209

Performing a combined weighting of the first clusters for determining a first photoplethysmography signal with mitigated ore removed flicker signals

Acquiring a second series of images of a subject at a second frame rate using the optical imaging system

Determining second clusters of pixels within the second series of images

Combining the second clusters pixel values for determining a second photoplethysmography signal

Deriving vital sign information of the subject from the first photoplethysmography signal and/or second photoplethysmography signal

Fig. 2

EP 4 410 189 A1

**Description**

FIELD OF THE INVENTION

The invention relates to camera-based photoplethysmography systems, in particular to identification and mitigation of flicker mimicking photoplethysmography (PPG) signal.

BACKGROUND OF THE INVENTION

Remote photoplethysmography (rPPG) is an optical measurement technique for detecting minute blood volume variations in cutaneous microcirculation using a digital camera. It allows noncontact measurements of various physiological parameters such as pulse rate (PR) and its variability, blood pressure, pulse transit time, etc. Remote photoplethysmography can be used to measure the cardiac pulse rate and the current cardiac phase in real-time based on a video stream of the face of a human subject. This technology has a wide range of potential applications. However, this rPPG measurement may be affected by the environment in which they are performed.

SUMMARY OF THE INVENTION

The invention provides for a system, a computer program, and a method in the independent claims. Embodiments are given in the dependent claims.

Remote detection of cardiac signals with camera-based photoplethysmography (PPG) has a wide range of applications also beyond the medical world. Modern ambient lighting as with LEDs commonly applies high-frequency modulation which may alias into the cardiac frequency band. The resulting flicker will disturb PPG detection. Embodiments may vary the camera frame rate slightly to identify the aliasing spectral component by the induced characteristic shift of this component. Embodiments are disclosed to shift it out of the cardiac band and to localize the modulated light sources.

In one aspect the invention provides for a system that comprises a memory storing machine executable instructions, and a processor, wherein execution of the machine executable instructions causes the processor to: acquire a first series of images of a subject using the optical imaging system, wherein the first series of images are acquired at a first frame rate; determine first clusters of pixels within the first series of images;

determining that a flicker signal is present in one or more of the first clusters of pixels, performing at least one of:
a) performing a combined weighting of the first clusters pixel values for determining a first photoplethysmography signal, wherein the weighting is performed to mitigate or remove a contribution of the flicker signal; or b): acquiring a second series of images with a second frame rate in order to prevent the flicker signal; determining second clusters of pixels within the second series of images; and combining the second clusters pixel values for determining a second photoplethysmography signal; and

derive vital sign information of the subject from the first photoplethysmography signal and/or second photoplethysmography signal.

The system may, for example, be a medical system.

The first series of images and the second series of images may be acquired concurrently. The concurrent acquisition may be performed using multiple cameras. Alternatively, the first series of images and the second series of images may be acquired consecutively.

For example, the performing of the at least one of a) or b) is performed in response determining that the flicker signal is present in the one or more of the first clusters of pixels.

The optical imaging system may be any imaging device capable of acquiring a sequence of images, and preferably is one or more digital video cameras that output digital video frames with a time stamp annotating each frame. The image acquired by the camera may be a video frame. The optical imaging system may be remote from the subject. This may enable to measure vital sign information remotely e.g., without having to attach a sensor to the subject. For example, the optical imaging system may be configured to acquire the first series of images from an imaging zone of the subject. The imaging zone may, for example, be the face of the subject. The first series of images may form a video. The first series of images comprises a sequence to images acquired at successive equally spaced points in time. The first series of images may be acquired at the first frame rate $f$. The frame rate refers to the number of frames per second. The optical imaging system may be configured to store and transmit the first series of images.

A light or illumination source may, for example, be provided in order to illuminate the imaging zone of the subject. For example, the illumination may be an infrared light or any visible light. The use of the light source may be beneficial because it may provide for a better operation of the optical imaging system and a more accurate determination of the vital sign information. The light source may, for example, be a light emitting diode (LED) or array of LEDs, an incandescent lamp, a halogen lamp, or so forth. The light source may or may not be part of the optical imaging system.

A repetitive change in magnitude over time, or modulation, of a luminous flux of the light source may occur. This modulation may have a frequency $f_L$ higher than the first frame rate $f$. However, this may induce flicker signals in the first series of images. For example, the optical imaging system may be capturing a video frame $n$ times a second (e.g., $f = n/s$) but lights of the light source may be flickering $n_L$ times a second (e.g., $f_L = n_L/s$). The optical imaging system may thus capture an image at a time when the light is not fully lit. This may cause a flicker in the image.

The present subject matter may advantageously process the first series of images using the first clusters in order to prevent the flickers. In one example, each first cluster of the first clusters may represent a distinct region of interest (ROI) of the imaging zone. Using different ROIs may enable to cover different features of the imaged zone and thus obtain accurate predictions of the vital sign information. Alternatively, the first clusters may represent one ROI. In this case, the first clusters may be randomly chosen within the image. Each first cluster of the first clusters may comprise a respective set of pixels in each image of the first series of images.

The present subject matter may determine the first photoplethysmography signal using the first clusters and/or determine the second photoplethysmography signal using the second clusters. The first photoplethysmography signal may be obtained by performing a weighted sum of the first clusters pixel values. The weighted sum may be performed in order to mitigate or completely remove the flicker that is detected in the one or more first clusters. The second photoplethysmography signal may be obtained by using new clusters which are obtained using the second frame rate $f''$. The first photoplethysmography signal and second photoplethysmography signal may be remote photoplethysmography signals.

In case only the first photoplethysmography signal is determined by the medical system, the vital sign information may be derived from the first photoplethysmography signal. In case only the second photoplethysmography signal is determined by the medical system, the vital sign information may be derived from the second photoplethysmography signal.

In case both the first photoplethysmography signal and the second photoplethysmography signal are determined by the medical system, the vital sign information may be derived from the first photoplethysmography signal and/or the second photoplethysmography signal.

For example, the vital sign information may be determined using the first photoplethysmography signal and the second photoplethysmography signal by combining (e.g., averaging) the first photoplethysmography signal and the second photoplethysmography signal and using the resulting combined photoplethysmography signal to derive the vital sign information. Alternatively, a first vital sign information may be derived from the first photoplethysmography signal, a second vital sign information may be derived from the second photoplethysmography signal and the first and second vital information may be combined (e.g., averaged) to obtain the vital sign information.

The present subject matter may thus provide accurate vital sign information. The derived vital sign information may advantageously be used for different applications. For example, the vital sign information may be used to monitor health status of a patient in a waiting room. In another example, the vital sign information may be used to monitor health status of a car driver. In another example, the vital sign information may be used to acquire cardiac magnetic resonance imaging (MRI) data.

According to one embodiment, the acquisition of the second series of images is performed in order to shift the flicker signal outside a cardiac frequency band. According to one embodiment, the execution of the machine executable instructions causes the processor to apply a band pass filter, that passes frequencies within the cardiac frequency band and rejects frequencies outside the cardiac frequency band, to obtain a filtered second photoplethysmography signal and to derive the vital sign information from the filtered signal.

According to one embodiment, the second frame rate $f''$ is obtained by shifting the first frame rate $f$ by an epsilon shift $\varepsilon$. For example, the first frame rate may be shifted as follows: $f'' = f \pm \varepsilon$, where $\varepsilon \le 0.02 \cdot f$.

An example implementation of this embodiment may consist of repeatedly shifting the frame rate with different values of the epsilon shift $\varepsilon$ until obtaining the flicker signal outside the cardiac frequency band. For example, if an LED driver modulates the light source at $f_L = 1002$ Hz and the first frame rate is $f = 20$ Hz, then the aliased frequency may be $f_A = 2$ Hz = 120 beats per minute (bpm), which is within the cardiac frequency band. If the first frame rate is changed to $f' = 20.01$ Hz, then the aliased frequency may be $$f'_A = 1.5 \text{ Hz} = 90 \text{ bpm}$$, which is still within the cardiac frequency band. If the frame rate is further changed to $f'' = 19.9$ Hz, then the aliased frequency may be $$f''_A = 7 \text{ Hz} = 420 \text{ bpm}$$, which is outside the cardiac frequency band. This may particularly be advantageous as any true PPG signal may remain after consecutive acquisitions at the same frequency so that the true PPG signal and the flicker component can be distinguished. In some cases, the heart rate may change between two consecutive acquisitions. In this case, the change in the frequency rate from one acquisition to another acquisition may be performed such that the expected shift of the flicker is much larger than any change of the heart rate that can be expected between two acquisitions. Alternatively, two cameras may be used to simultaneously or concurrently acquire the first series of images and the second series of images at two different frame rates e.g., at $f$ and $f'$ or at $f'$ and $f''$ respectively.

According to one embodiment, the flicker signal is caused by a modulation of a luminous flux of a light source, wherein the modulation has a modulation frequency $f_L$ that is defined as function of the first frame rate $f$ as follows:

$$\frac{f_L}{f} = \alpha + \beta$$

, with $\alpha \in N$ and $-0.5 \leq \beta < 0.5$, wherein the frequency of the flicker signal is defined as $f_A = |\beta| f$, wherein

the second frame rate $f''$ is defined as follows: $f'' = \frac{f_L - f_A''}{\alpha} = \frac{\alpha f + \beta f - f_A''}{\alpha} = \frac{\alpha f + f_A - f_A''}{\alpha} = f + (f' - f)\frac{f_A - f_A''}{f_A - f_A'}$ ,

$$f'' = \frac{f_L - f_A''}{\alpha} = \frac{\alpha f + \beta f - f_A''}{\alpha} = \frac{\alpha f + f_A - f_A''}{\alpha} = f + (f' - f)\frac{f_A - f_A''}{f_A - f_A'}$$

where $f'$ is an intermediate frame rate, $f_A'$ , is the frequency of the flicker signal obtained for the intermediate frame rate $f_A'$ , where $f_A''$ is the desired frequency of the flicker signal associated with the second frame rate $f''$. This embodiment may enable an accurate and systematic method for deriving the second frame rates.

According to one embodiment, the combined weighting is performed using weights, wherein the combined weighting is performed for ignoring the one or more first clusters using null weights or down weighting the one or more first clusters using weights having lower values compared to the other weights.

According to one embodiment, the combining of second cluster pixel values comprises: for each image in the second series of images: determining an individual photoplethysmography signal per second cluster using pixel values of the second cluster and combining the individual photoplethysmography signals.

For example, the determined second clusters may be $N2$ second clusters, $cl_1^2 \ldots cl_{N2}^2$ respectively, where $N2 \geq 2$. The photoplethysmography signal value of a given image of the second series of images may, for example, be obtained as follows: $\frac{\sum_{k=1}^{N2} Av_k}{N2}$ , where $Av_k$ is the average of pixel values or the individual photoplethysmography signal value of cluster $cl_k^2$ in the given image.

According to one embodiment, the combined weighting of first cluster pixel values comprises: for each image in the first series of images: determining an individual photoplethysmography signal per first cluster using pixel values of the first cluster and performing a combined weighting the individual photoplethysmography signals using respective weights.

For example, the determined first clusters may be $N1$ first clusters, $cl_1^1 \ldots cl_{N1}^1$ respectively, where $N1 \geq 2$.

The first clusters $cl_1^1 \ldots cl_{N1}^1$ may be associated with weights $w_1^1 \ldots w_{N1}^1$ with $0 \leq w_j^1 < 1$ respectively. Each first cluster $cl_j^1$ of the one or more first clusters which have the flicker signal may be assigned a null weight $w_j^1 = 0$ in order to remove the flicker signal, where $j$ is an index referring to the one or more first clusters. Alternatively, each first cluster $cl_j^1$ of the one or more first clusters which have the flicker signal may be assigned a weight $w_j^1 < w_i^1$ that is smaller than the weights of the other first clusters $cl_i^1$ in order to mitigate the contribution of the flicker signal into the first photoplethysmography signal, where $i$ is an index referring to the clusters which have no flicker signal. The photoplethysmography signal value of a given image of the first series of images may, for example, be obtained as follows:

$\frac{\sum_{k=1}^{N1} w_k^1 \cdot Av_k}{\sum_{k=1}^{N1} w_k^1 \cdot}$ , where $Av_k$ is the average of pixel values or the individual photoplethysmography signal value of cluster

$cl_k^1$ in the given image.

According to one embodiment, the first clusters are different from or same as the second clusters. Having the same clusters for the first and second acquisitions may enable a consistent combination of the first and second photoplethysmography signals for obtaining the vital sign information. Having different clusters for the first and second acquisitions may cover different features of the acquired images and thus provide an accurate estimation of the vital sign information.

According to one embodiment, each first cluster of the first clusters comprises a same set of pixels in each image of the first series of images. This embodiment may be advantageous because the clustering may save processing resources compared to an adjustable/dynamic clustering. This embodiment may particularly be advantageous in case the imaging zone is not moving during the acquisition of the first series of images.

According to one embodiment, each cluster of the first clusters comprises different sets of pixels in two or more images of the first series of images. This clustering may be advantageous in case the imaged zone can move during the acquisition of the first series of images. The movement of the imaged zone may be tracked over the sequence of images in order to adapt the clusters per image. For example, in case the imaging zone is the face of the subject, a face tracker may be used for tracking the movement of the subject's face and to readjust the cluster from image to image. An advantage of this embodiment may reside in providing improved vital sign information even when the subject is moving during the acquisition of the first series of images.

According to one embodiment, the optical imaging system is configured for imaging a skin surface of the subject. This embodiment may be beneficial because the optical imaging system is imaging directly a surface of the subject.

According to one embodiment, the optical imaging system comprises one or more video cameras. If the optical imaging system comprises multiple cameras, these cameras may acquire video data from the same imaging zone and may be operated at same frame rates or slightly different frame rates to simultaneously acquire the video data.

For example, the first series of images may be divided into subsets of images, each subset being a acquired by a respective camera. An individual first photoplethysmography signal may be obtained per subset of images. The individual first photoplethysmography signals may be combined (e.g., averaged) to obtain the first photoplethysmography signal. This may particularly be advantageous if the same frame rate is used by the cameras.

The second series of images may be split into subsets of images. An individual second photoplethysmography signal may be obtained per subset. The individual second photoplethysmography signals may be combined to obtain the second photoplethysmography signal. This may particularly be advantageous if the same frame rate is used by the cameras. Alternatively, the cameras may acquire the first series of images and the second series of images using slightly different frame rates, e.g., the first frame rate and second frame rate. This may be advantageous as a derived heart rate may not change between the two acquisitions while the position of the flicker signal changes.

According to one embodiment, the medical system further comprises a magnetic resonance imaging system, wherein the memory further contains pulse sequence commands configured to control the magnetic resonance imaging system to acquire k-space data according to the magnetic resonance imaging protocol, wherein execution of the machine executable instructions further causes the computational system to: acquire the k-space data by controlling the magnetic resonance imaging system in accordance with the determined vital sign information. This embodiment may be advantageous as it may enable an accurate acquisition of MR images. For example, this may enable to acquire high quality cardiac MRI data by synchronizing the image acquisition with a specific time during the cardiac cycle of the subject. The vital sign information may, for example, be used to detect the largest and steepest peak in the cardiac cycle in order to trigger the acquisition of parts of the k-space.

According to one embodiment, the vital sign information comprises at least one of: heart rate, respiration rate, blood pressure, pulse transit time and real-time triggers.

In another aspect the invention relates to a computer program comprising machine executable instructions for execution by a computational system; wherein execution of the machine executable instructions causes the computational system to: acquire a first series of images of a subject using an optical imaging system, wherein the first series of images are acquired at a first frame rate; determine first clusters of pixels within the first series of images; determining that a flicker signal is present in one or more of the first clusters of pixels, performing at least one of: performing a combined weighting of the first clusters pixel values for determining a first photoplethysmography signal, wherein the weighting is performed to mitigate or remove a contribution of the flicker signal; or acquiring a second series of images with a second frame rate in order to prevent the flicker signal; determining second clusters of pixels within the second series of images; and combining the second clusters pixel values for determining a second photoplethysmography signal; and derive vital sign information of the subject from the first photoplethysmography signal and/or second photoplethysmography signal.

In another aspect the invention relates to a method comprising: acquiring a first series of images of a subject using an optical imaging system, wherein the first series of images are acquired at a first frame rate; determining first clusters of pixels within the first series of images; determining that a flicker signal is present in one or more of the first clusters of pixels, performing at least one of: performing a combined weighting of the first clusters pixel values for determining a first photoplethysmography signal, wherein the weighting is performed to mitigate or remove a contribution of the flicker signal; or acquiring a second series of images with a second frame rate in order to prevent the flicker signal; determining second clusters of pixels within the second series of images; and combining the second clusters pixel values for determining a second photoplethysmography signal; and deriving vital sign information of the subject from the first

photoplethysmography signal and/or second photoplethysmography signal.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance image or MR image is defined herein as being the reconstructed two- or three-dimensional visualization of anatomic data contained within the k-space data. This visualization can be performed using a computer.

BRIEF DESCRIPTION OF THE DRAWINGS

In the following preferred embodiments of the invention will be described, by way of example only, and with reference to the drawings in which:

Fig. 1 illustrates an example of a medical system;
Fig. 2 shows a flowchart of a method of using the medical system of Fig. 1;

Fig. 3 shows a flowchart of a method of using the medical system of Fig. 1;
Figs. 4a-b shows a flowchart of a method of using the medical system of Fig. 1;
Fig. 5 illustrates an example of a medical system;
Fig. 6 shows a flowchart of a method of using the medical system of Fig. 5; and
Figs. 7a-b show plots representing flicker signals.

DETAILED DESCRIPTION OF EMBODIMENTS

Like numbered elements in these figures are either equivalent elements or perform the same function. Elements which have been discussed previously will not necessarily be discussed in later figures if the function is equivalent.

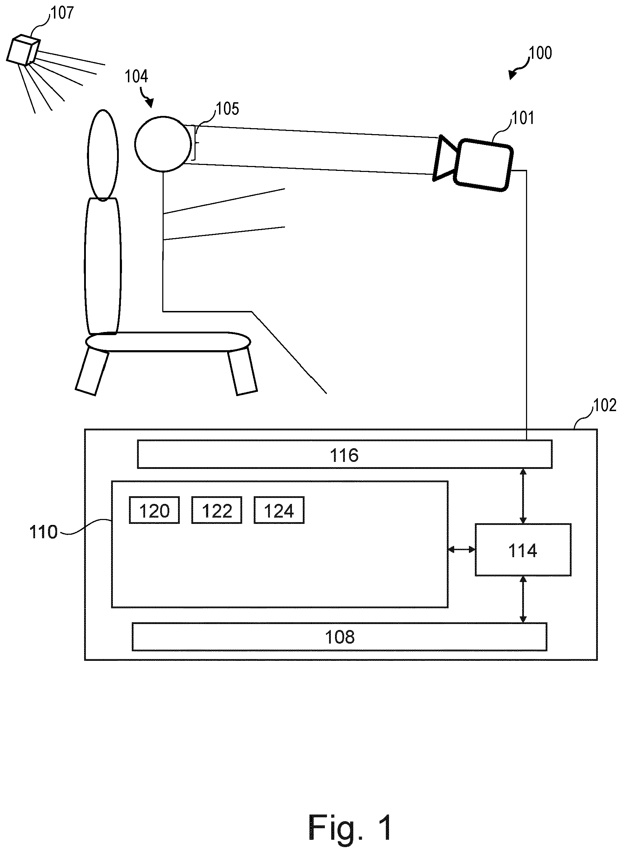

Fig. 1 shows an example of a medical system 100. There is a subject 104 reposing on a seat. A camera 101 is focused on an imaging zone 105 of the subject 104. The camera 101 is connected to a hardware interface 116 of a computer system 102. The camera 101 may be configured to acquire video data from the imaging zone 105, to store the video data and transmit the video data to the computer system 102. The video data may, for example, be transmitted as a stream. For example, the computer system 102 may control the acquisition of the video data by the camera 101. A light source 107 is provided in order to illuminate the subject 104 and in particular the imaging zone 105.

The computer system 102 is provided with a processor 114. The computer system 102 is intended to represent one or more computers or computer systems. The processor 104 is intended to represent one or more computational systems or computational cores. The computer system 102 is further shown as containing the hardware interface 116 which is connected to the processor 114. If other components of the medical system 100 are present or included such as a magnetic resonance imaging system, then the hardware interface 116 could be used to exchange data and commands with these other components. The computer system 102 is further shown as comprising an optional user interface 108 which may provide various means for relaying data and receiving data and commands from an operator.

The computer system 102 is further shown as comprising a memory 110 that is connected to the processor 114. The memory 110 is intended to represent various types of memory which may be accessible to the processor 114. The memory 110 is shown as containing machine-executable instructions 120. The machine-executable instructions 120 are instructions which enable the processor 114 to perform various control, data processing, and image processing tasks. The memory 110 is further shown as containing vital sign information 122 obtained from videos 124 acquired by the camera 101.

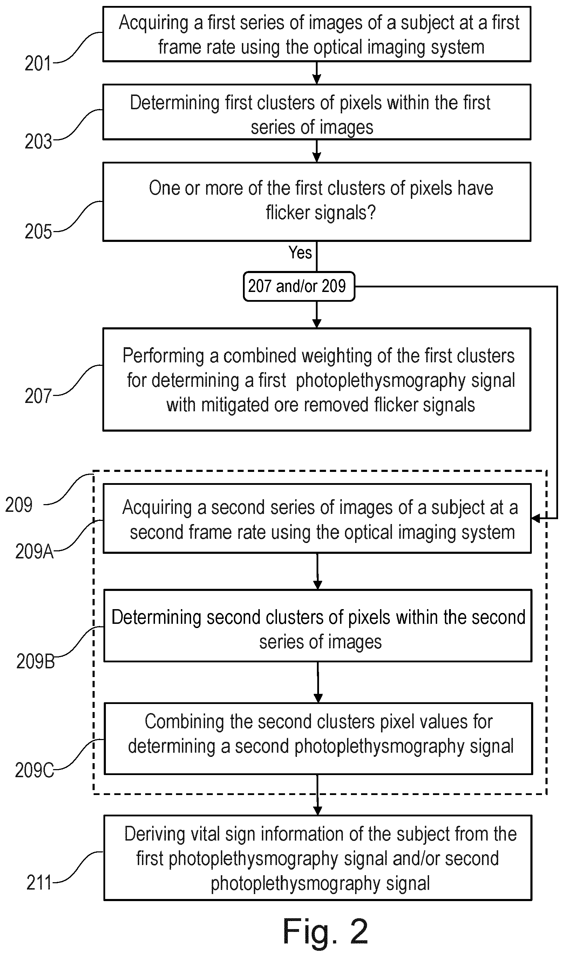

The medical system 100 may be used to derive rPPG signals. However, the use of rPPG signals may be hampered by limited PPG signal amplitude and signal disturbances. In particular, a flicker due to modulated ambient lighting provided by the light source 107 may disturb PPG signal detection or mimic a PPG signal. Indeed, the light source 107 may be modulated at high frequencies which are not directly perceivable by the human eye. When the camera 105 acquires the PPG raw data with a fixed frame rate, which is smaller than the modulation frequency, then an interference of light modulation and frame rate may lead to aliasing into the expected range of cardiac frequencies of about 0.5 Hz to 3 Hz. In addition, applying a bandpass filter in the cardiac frequency band may not be enough to get rid of flicker contributions. Thus, the present subject matter may use the medical system 100 to perform a method such as the method of Fig. 2 in order to prevent flickers in the rPPG signals.

Fig. 2 is a flowchart of a method for determining vital sign information of a subject in accordance with an example of the present subject matter. The method may for example be performed by the medical system of Fig. 1.

A first series of images of the subject may be acquired in step 201 from an imaging zone of the subject using an optical imaging system such as the camera 101. The first series of images are acquired at a first frame rate.

First clusters of pixels may be determined in step 203 within the first series of images.

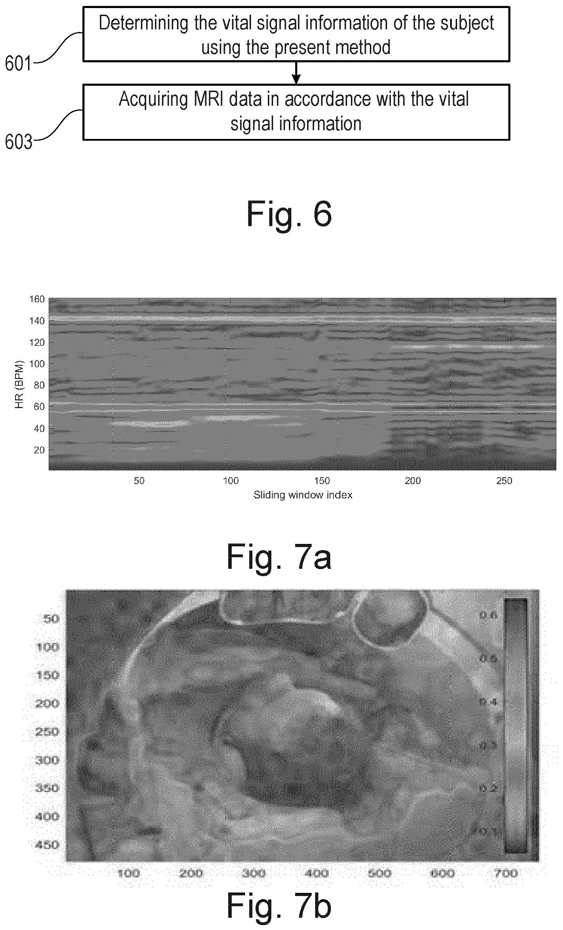

It may be determined in step 205 whether a flicker signal is present in one or more of the first clusters of pixels. Fig. 7a shows an example flicker signal.

In response to determining that a flicker signal is present in one or more of the first clusters of pixels, at least one of step 207 and step 209 may be performed.

In step 207, a combined weighting of the first clusters pixel values may be performed in step 207 in order to determine a first photoplethysmography signal, wherein the weighting is performed to mitigate or remove a contribution of the flicker signal.

Step 209 comprises steps 209A to 209C.

In step 209A, a second series of images may be acquired from the imaging zone of the subject with a second frame rate in order to prevent the flicker signal. Second clusters of pixels may be determined in step 209B within the second series of images. The second clusters pixel values may be combined in step 209C for determining a second photoplethysmography signal.

Vital sign information of the subject may be derived in step 211 from the first photoplethysmography signal and/or second photoplethysmography signal.

In one example, steps 201 to 211 may be repeated on a periodic basis e.g., in intervals determined by typical time scales in which ambient lighting changes, e.g., every few minutes, or in response to a change in ambient lighting level, or in response to receiving a request of vital sign information.

In one example alternative implementation of the method Fig. 2, the acquisition step 209A may be performed concurrently or simultaneously with the acquisition step 201 e.g., using multiple cameras. In this case, step 209 may consist of sub-steps 209B to 209C.

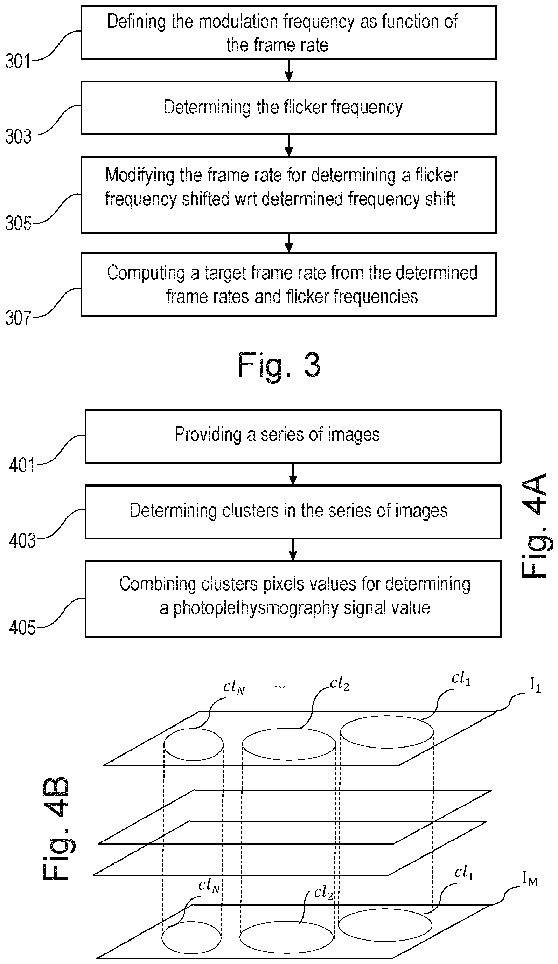

Fig. 3 is a flowchart of a method for determining a frame rate $f''$ in accordance with an example of the present subject matter. The method may for example be performed by the medical system of Fig. 1. The method of Fig. 3 may for example be used for performing the acquisition step 209A of Fig. 2.

For example, the light source 107 may be modulated at a frequency $f_L$ and first series of images may be recorded by the camera 101 with a frame rate $f$. The modulation frequency $f_L$ may be defined in step 301 as function of the frame rate $f$ of the camera as follows:

$$\frac{f_L}{f} = \alpha + \beta$$

, with $\alpha \in N$, $-0.5 \le \beta < 0.5$.

The aliased or flicker frequency $f_A$ of the light source in the video stream may be determined in step 303 by the following equation $f_A = |\beta|f$. For example, if an LED driver modulates the light source at modulation frequency $f_L = 1002$ Hz and the frame rate is $f = 20$ Hz, then the aliased frequency is $f_A = 2$ Hz $= 120$ bpm, which is within the cardiac frequency band. This may be solved by shifting the aliased frequency outside the cardiac frequency band using a new frame rate $f''$. However, as the modulation frequency $f_L$ of the light source is not known, it may be difficult to calculate the frame rate $f''$ that results in a suitable aliasing frequency $f_A''$ outside the cardiac band based on a measurement using a single frame rate $f$.

For that, the frame rate may be modified in step 305 only slightly from $f$ to $f'$ and a respective pair of aliasing frequencies $f_A$ and $f_A'$ is derived by identification of the spectral peak that changes position. In addition, the above equation can be used to calculate $\alpha$ and $f_L$. This may result in: $\frac{f_L}{f} = \alpha + \beta$ and $\frac{f_L}{f'} = \alpha' + \beta'$. This calculation assumes that a small change in frame rate does only change $\beta$ whereas the integer part $\alpha$ remains unchanged (i.e., $\alpha = \alpha'$): $f_L = (\alpha + \beta)f = (\alpha' + \beta')f'$. Since $\alpha = \alpha'$, this may result in $\alpha = \frac{\beta f - \beta' f'}{f' - f} = \frac{f_A - f_A''}{f' - f}$.

With the same assumption, a new target frame rate $f''$ may be calculated in step 307 which shifts the flicker to some new frequency $f_A''$ as follows:

$$f'' = \frac{f_L - f_A''}{\alpha} = \frac{\alpha f + \beta f - f_A''}{\alpha} = \frac{\alpha f + f_A - f_A''}{\alpha} = f + (f' - f)\frac{f_A - f_A''}{f_A - f_A'}$$

which expresses the new fame rate $f''$ in terms of $f$, $f'$, $f_A$, $f_A'$, and $f_A''$. Continuing with the above numerical example one may choose a target flicker frequency $f_A'' = 7$ Hz $= 420$ bpm, which according to the above equation requires a frame rate $f'' = 19.9$ Hz.

Fig. 4A is a flowchart of a method for determining a photoplethysmography signal in accordance with an example of the present subject matter. The method may for example be performed by the medical system of Fig. 1.

A series of images may be provided in step 401. Fig. 4B depicts a series of M images, $I_1...I_M$. For example, the series of images $I_1...I_M$ may be the first series of images acquired in step 201 or second series of images acquired in step 209A of Fig. 2

A clustering may be performed in step 403 in order to determine clusters in the series of images. For example, different ROIs may be located in the imaging zone and for each ROI, a cluster may be created in step 403. The ROIs may, for example, cover different parts of the face of the subject 104. In this example, the ROIs do not overlap but it is not limited to as the present subject matter may be used for partially or fully overlapping ROIs. Fig. 4B depicts a number $N$ of clusters, $cl_1$, $cl_2$, ..., $cl_N$. Each cluster $cl_i$ has its own set of $m_i$ pixels $px_1^i...px_{m_i}^i$, where $i$ varies between 1 and $N$.

For each image of the images $I_1...I_M$, step 405 may be performed. In step 405, the clusters pixel values present in the image may be combined in step 405 for determining a photoplethysmography signal value. This may result in a set of $M$ photoplethysmography signal values $PPG_1...PPG_M$ which form the photoplethysmography signal. The present subject matter may provide alternative techniques for performing step 405.

In one first example, for a current $k^{th}$ image $I_k$, the photoplethysmography signal value $PPG_k$ may be computed cluster wise. Each cluster of the clusters in the image $I_k$ may be processed in order to compute the average of pixel values of the cluster. For each $i^{th}$ cluster, the average $Av_i$ may be defined as follows: $Av_i = \frac{\sum_{l=1}^{m_i} px_l^i}{m_i}$ . And the photoplethysmography signal value may be defined as follows: $PPG_k = \frac{\sum_{i=1}^{N} Av_i}{N}$ .

In one second example, for a current $k^{th}$ image $I_k$, the photoplethysmography signal value may be obtained pixel wise. It may be defined as follows: $PPG_k = \frac{\sum_{l=1}^{m_T} px_l^i}{m_T}$ , where $m_T$ is the total number of pixels in the clusters of the image $I_k$, $m_T = \sum_{i=1}^{N} m_i$ .

In one third example, for a current $k^{th}$ image $I_k$, the photoplethysmography signal value $PPG_k$ may be computed cluster wise. Each cluster of the clusters in the image $I_k$ may be processed in order to compute the average of pixel values of the cluster. For each $i^{th}$ cluster, the average $Av_i$ may be defined as follows: $Av_i = \frac{\sum_{l=1}^{m_i} px_l^i}{m_i}$ . And the photoplethysmography signal value may be defined as a weighted sum of the averages: $PPG_k = \frac{\sum_{i=1}^{N} w_i \cdot Av_i}{\sum_{i=1}^{N} w_i}$ , where $w_i$ is the weight assigned to the $i^{th}$ cluster. Using the weights may enable to down weight or ignore some clusters e.g., $w_i$ may be set to zero to ignore the $i^{th}$ cluster or set to a value that is smaller than the remaining weights to down weight the $i^{th}$ cluster. This weighted sum may, for example, be used for performing step 207 of Fig. 2.

In one fourth example, for a current $k^{th}$ image $I_k$, the photoplethysmography signal value may be obtained pixel wise. It may be defined as follows: $PPG_k = \frac{\sum_{l=1}^{m_T} w_l \cdot px_l^i}{m_T}$ , where $m_T$ is the total number of pixels in the clusters of the image $I_k$, $m_T = \sum_{i=1}^{N} m_i$ , and $w_l$ is the weight assigned to the $l^{th}$ pixel. For example, the same weight may be assigned to the pixels of the same cluster. The weight $w_l$ may be set to zero to ignore the $l^{th}$ pixel or set to a value that is smaller than the remaining weights to down weight the $l^{th}$ pixel.

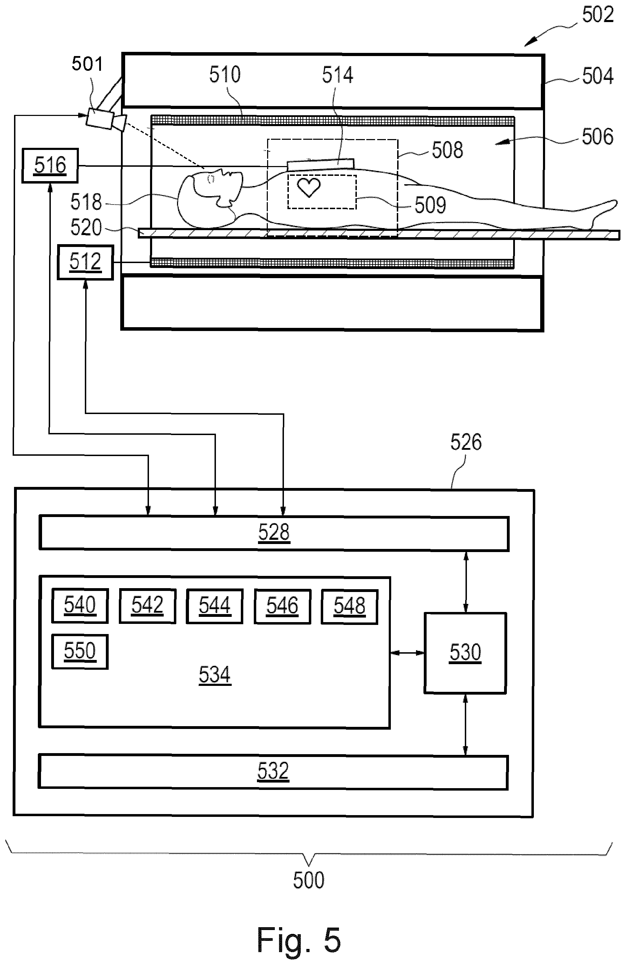

Fig. 5 shows a further example of a medical instrument 500. In this example the medical instrument 500 comprises a magnetic resonance imaging system 502. The magnetic resonance imaging system 502 comprises a magnet 504. The magnet 504 is a superconducting cylindrical type magnet with a bore 506 through it. The use of different types of magnets is also possible; for instance, it is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 506 of the cylindrical magnet 504 there is an imaging volume 508 where the magnetic field is strong and uniform enough to perform magnetic resonance imaging. A region of interest 509 is shown within the imaging volume 508. A subject 518 is shown as being supported by a subject support 520 such that at least a portion of the subject 518 is within the imaging volume 508 and the region of interest 509.

Within the bore 506 of the magnet there is also a set of magnetic field gradient coils 510 which is used for acquisition of magnetic resonance data to spatially encode magnetic spins within the imaging volume 508 of the magnet 504. The magnetic field gradient coils 510 connected to a magnetic field gradient coil power supply 512. The magnetic field gradient coils 510 are intended to be representative. Typically, magnetic field gradient coils 510 contain three separate sets of coils for spatially encoding in three orthogonal spatial directions. A magnetic field gradient power supply supplies current to the magnetic field gradient coils. The current supplied to the magnetic field gradient coils 510 is controlled as a function of time and may be ramped or pulsed.

Adjacent to the imaging volume 508 is a radio-frequency coil or surface coil 514 for manipulating the orientations of magnetic spins within the imaging volume 508 and for receiving radio transmissions from spins also within the imaging volume 508. The radio frequency antenna may contain multiple coil elements. The radio frequency antenna may also be referred to as a channel or antenna. The radio-frequency coil 514 is connected to a radio frequency transceiver 516. The radio-frequency coil 514 and radio frequency transceiver 516 may be replaced by separate transmit and receive coils and a separate transmitter and receiver. It is understood that the radio-frequency coil 514 and the radio frequency transceiver 516 are representative. The radio-frequency coil 514 is intended to also represent a dedicated transmit antenna and a dedicated receive antenna. Likewise, the transceiver 516 may also represent a separate transmitter and receivers. The radio-frequency coil 514 may also have multiple receive/transmit elements and the radio frequency transceiver 516 may have multiple receive/transmit channels. For example, if a parallel imaging technique such as SENSE is performed, the radio-frequency could 514 will have multiple coil elements.

In this example a camera 501 can be seen as being mounted on the flange of the magnet 504. The face of the subject is imaged by the camera 501.

The transceiver 516, the gradient controller 112, and camera 106 are shown as being connected to a hardware interface 528 of a computer system 526. The computer system further comprises a processor 530 that is in communication with the hardware system 528, a memory 534, and a user interface 532. The memory 534 may be any combination of memory which is accessible to the processor 530. This may include such things as main memory, cached memory, and also non-volatile memory such as flash RAM, hard drives, or other storage devices. In some examples the memory 530 may be considered to be a non-transitory computer-readable medium.

The computer memory 534 is shown as containing machine-executable instructions 540. The machine-executable instructions contain commands or instructions which enable the processor 530 to control the operation and function of the magnetic resonance imaging system 502. The computer memory 534 is shown as further containing pulse sequence commands 542. The pulse sequence commands 542 are either instructions or data which may be converted into instructions which enable the processor 530 to control the magnetic resonance imaging system 502 to acquire magnetic resonance data.

The computer memory 534 is shown as containing a series of images 544 acquired with the camera 501. The various images in the series of images 544 can be used to derive vital signal information 546 which is also shown as being stored in the memory 534. For example, the method illustrated in Fig. 2, could be performed repeatedly to generate the vital sign information 546 which can be used to trigger the acquisition of magnetic resonance data 548 using the pulse sequence commands 542. The magnetic resonance data 548 can then be reconstructed into a magnetic resonance image 550.

Fig. 6 is a flowchart of a method for acquiring MRI data in accordance with an example of the present subject matter. The method may for example be performed by the medical system of Fig. 5.

In step 601, the method Figure 2 may be performed in order to determine the vital signal information of the subject.

The MRI system may be controlled to acquire in step 603 MRI data in accordance with the vital signal information. For example, this may enable to acquire high quality cardiac MRI data by synchronizing the image acquisition with a specific time during the cardiac cycle of the subject. The vital sign information may, for example, be used to detect the largest and steepest peak in the cardiac cycle or other features of the vital signal in order to trigger the acquisition of parts of the k-space. Steps 601 and 603 may be repeated for many cardiac cycles to acquire MRI data for one MRI scan, e.g., a single MR image or a set of MR images.

Fig. 7a shows the measured heart rate (vertical axis) as function of acquisition time (horizontal axis) representing a true PPG signal at 60 bpm and a flicker-based signal at 140 bpm. A gray based color-overlay of the amplitude of the flicker-based signal at 140 bpm is shown in Fig. 7b. The colorbar indicates normalized values of the amplitude of the signal. This plot indicates that the flicker signal does not originate from a human skin but from the bore wall of the MR system visible in the upper left corner of the image, which is likely a reflection of flickering ambient lighting at that part of the bore wall.

**Claims**

1. A system comprising:

   an optical imaging system configured for acquiring a series of optical images;
   a memory for storing machine executable instructions;
   a processor for controlling the system, wherein execution of the machine executable instructions causes the processor to:

acquire a first series of images of a subject using the optical imaging system, wherein the first series of images are acquired at a first frame rate;
determine first clusters of pixels within the first series of images;
determining that a flicker signal is present in one or more of the first clusters of pixels;
performing at least one of:

performing a combined weighting of the first clusters pixel values for determining a first photoplethysmography signal, wherein the weighting is performed to mitigate or remove a contribution of the flicker signal; or
acquiring a second series of images with a second frame rate in order to prevent the flicker signal; determining second clusters of pixels within the second series of images; and combining the second clusters pixel values for determining a second photoplethysmography signal;

derive vital sign information of the subject from the first
photoplethysmography signal and/or second photoplethysmography signal.

2. The system of claim 1, wherein the acquiring of the second series of images is performed in order to shift the flicker signal outside a cardiac frequency band.

3. The system of claim 1 or 2, wherein the second frame rate is obtained by shifting the first frame rate by an epsilon shift.

4. The system of any of the preceding claims, the flicker signal being caused by a modulation of a luminous flux of a light source, wherein the modulation has a modulation frequency $f_L$ that is defined as function of the first frame rate $f$ as follows: $\frac{f_L}{f} = \alpha + \beta$, with $\alpha \in N$ and $-0.5 \le \beta < 0.5$, wherein the frequency of the flicker signal is defined as $f_A = |\beta|f$, wherein the second frame rate $f''$ is defined as follows:

$$f'' = \frac{f_L - f_A''}{\alpha} = \frac{\alpha f + \beta f - f_A''}{\alpha} = \frac{\alpha f + f_A - f_A''}{\alpha} = f + (f' - f)\frac{f_A - f_A''}{f_A - f_A'}$$

, where $f'$ is an intermediate frame rate, $f_A'$ is the frequency of the flicker signal obtained for the intermediate frame rate $f_A'$, where $f_A''$ is the desired frequency of the flicker signal associated with the second frame rate $f''$.

5. The system of any of the preceding claims, wherein the combined weighting is performed using weights, wherein the combined weighting is performed for ignoring the one or more first clusters using null weights or down weighting the one or more first clusters using weights having lower values compared to the other weights.

6. The system of any of the preceding claims,

wherein the combining of second cluster pixel values comprises: for each image in the second series: determining an individual photoplethysmography signal per cluster using pixel values of the cluster and combining the individual photoplethysmography signals for obtaining the second photoplethysmography signal;
wherein the combined weighting of cluster pixel values comprises: for each image in the first series: determining an individual photoplethysmography signal per cluster using pixel values of the cluster and performing a combined weighting the individual photoplethysmography signals using respective weights for obtaining the first photoplethysmography signal.

7. The system of any of the preceding claims, wherein the first clusters are different from or same as the second clusters.

8. The system of any of the preceding claims, wherein each cluster of the first clusters comprises a same set of pixels in each image of the first series of images.

9. The system of any of the preceding claims 1 to 7, wherein each cluster of the first clusters comprises different sets of pixels in two or more images of the first series of images.

10. The system of any one of the preceding claims, wherein the optical imaging system is configured for imaging a skin surface of the subject.

11. The system of any one of claims, wherein the optical imaging system comprises multiple cameras.

12. The system of any one of claims, wherein the system further comprises a magnetic resonance imaging system, wherein the memory further contains pulse sequence commands configured to control the magnetic resonance imaging system to acquire k-space data according to the magnetic resonance imaging protocol, wherein execution of the machine executable instructions further causes the computational system to: acquire the k-space data by controlling the magnetic resonance imaging system in accordance with the determined vital sign information.

13. The system of any one of claims, the vital sign information comprising at least one of: heart rate, respiration rate and real-time triggers.

14. A computer program comprising machine executable instructions, wherein execution of the machine executable instructions causes a computational system to:

acquire a first series of images of a subject using an optical imaging system, wherein the first series of images are acquired at a first frame rate;
determine first clusters of pixels within the first series of images;
determining that a flicker signal is present in one or more of the first clusters of pixels;
performing at least one of: performing a combined weighting of the first clusters pixel values for determining a first photoplethysmography signal, wherein the weighting is performed to mitigate or remove a contribution of the flicker signal; or acquiring a second series of images with a second frame rate in order to prevent the flicker signal; determining second clusters of pixels within the second series of images; and combining the second clusters pixel values for determining a second photoplethysmography signal;
derive vital sign information of the subject from the first photoplethysmography signal and/or second photoplethysmography signal.

15. A method comprising:

acquiring a first series of images of a subject using an optical imaging system, wherein the first series of images are acquired at a first frame rate;
determining first clusters of pixels within the first series of images;
determining that a flicker signal is present in one or more of the first clusters of pixels;
performing at least one of:

performing a combined weighting of the first clusters pixel values for determining a first photoplethysmography signal, wherein the weighting is performed to mitigate or remove a contribution of the flicker signal;
or acquiring a second series of images with a second frame rate in order to prevent the flicker signal; determining second clusters of pixels within the second series of images; and combining the second clusters pixel values for determining a second photoplethysmography signal;
deriving vital sign information of the subject from the first photoplethysmography signal and/or second photoplethysmography signal.

Fig. 1

Fig. 2

| 301 | Defining the modulation frequency as function of the frame rate |
|---|---|

↓

| 303 | Determining the flicker frequency |
|---|---|

↓

| 305 | Modifying the frame rate for determining a flicker frequency shifted wrt determined frequency shift |
|---|---|

↓

| 307 | Computing a target frame rate from the determined frame rates and flicker frequencies |
|---|---|

## Fig. 3

| 401 | Providing a series of images |
|---|---|

↓

| 403 | Determining clusters in the series of images |
|---|---|

↓

| 405 | Combining clusters pixels values for determining a photoplethysmography signal value |
|---|---|

Fig. 4A

Fig. 4B

$cl_N$  ...  $cl_2$  $cl_1$  $I_1$

$cl_N$  $cl_2$  $cl_1$  $I_M$

Fig. 5

601 ⟍
| Determining the vital signal information of the subject using the present method |
| --- |

603 ⟍
| Acquiring MRI data in accordance with the vital signal information |
| --- |

## Fig. 6

## Fig. 7a

## Fig. 7b

| | Europäisches Patentamt European Patent Office Office européen des brevets | **EUROPEAN SEARCH REPORT** | Application Number EP 23 15 4691 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 207 862 A1 (COVIDIEN LP [US]) 23 August 2017 (2017-08-23) * paragraphs [0028], [0080], [0040] – [0047], [0050] – [0052], [0124], [0071] – [0073], [0064], [0059] * | 1–15 | INV. A61B5/024 A61B5/055 A61B5/00 |
| X | US 2022/054089 A1 (DE HAAN GERARD [NL] ET AL) 24 February 2022 (2022-02-24) * paragraphs [0014] – [0030] * | 1,14,15 | ADD. A61B5/1455 |
| X | EP 2 967 376 A1 (KONINKL PHILIPS NV [NL]) 20 January 2016 (2016-01-20) * paragraphs [0018] – [0023], [0038] * | 1,14,15 | |

TECHNICAL FIELDS
SEARCHED    (IPC)

A61B

The present search report has been drawn up for all claims

| Place of search | Date of completion of the search | Examiner |
|---|---|---|
| The Hague | 28 July 2023 | Kowalczyk, Szczepan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 4691

This annex lists the patent family members relating to the patent documents cited in the above-mentioned European search report.
The members are as contained in the European Patent Office EDP file on
The European Patent Office is in no way liable for these particulars which are merely given for the purpose of information.

28-07-2023

| Patent document cited in search report | | | Publication date | Patent family member(s) | | | Publication date |
|---|---|---|---|---|---|---|---|
| EP 3207862 | A1 | | 23-08-2017 | CA | 2958003 | A1 | 19-08-2017 |
| | | | | CA | 2958010 | A1 | 19-08-2017 |
| | | | | EP | 3207862 | A1 | 23-08-2017 |
| | | | | EP | 3207863 | A1 | 23-08-2017 |
| | | | | US | 2017238805 | A1 | 24-08-2017 |
| | | | | US | 2017238842 | A1 | 24-08-2017 |
| | | | | US | 2019307365 | A1 | 10-10-2019 |
| | | | | US | 2020268281 | A1 | 27-08-2020 |
| | | | | US | 2020289024 | A1 | 17-09-2020 |
| | | | | US | 2022211296 | A1 | 07-07-2022 |
| | | | | US | 2022257143 | A1 | 18-08-2022 |
| US 2022054089 | A1 | | 24-02-2022 | CN | 108471989 | A | 31-08-2018 |
| | | | | EP | 3402402 | A1 | 21-11-2018 |
| | | | | JP | 6849684 | B2 | 24-03-2021 |
| | | | | JP | 2019505263 | A | 28-02-2019 |
| | | | | US | 2019000391 | A1 | 03-01-2019 |
| | | | | US | 2022054089 | A1 | 24-02-2022 |
| | | | | WO | 2017121834 | A1 | 20-07-2017 |
| EP 2967376 | A1 | | 20-01-2016 | BR | 112015022112 | A2 | 18-07-2017 |
| | | | | CN | 105050492 | A | 11-11-2015 |
| | | | | EP | 2967376 | A1 | 20-01-2016 |
| | | | | JP | 6423807 | B2 | 14-11-2018 |
| | | | | JP | 2016514986 | A | 26-05-2016 |
| | | | | RU | 2015143949 | A | 20-04-2017 |
| | | | | US | 2014276089 | A1 | 18-09-2014 |
| | | | | WO | 2014140148 | A1 | 18-09-2014 |

EPO FORM P0459

For more details about this annex : see Official Journal of the European Patent Office, No. 12/82